# EUROPEAN PATENT SPECIFICATION

(11) **EP 2 670 320 B1**
(45) Date of publication and mention of the grant of the patent: **24.12.2014**
(21) Application number: 12703391.8
(22) Date of filing: 31.01.2012
(51) Int. Cl.: A61B 17/68

(54) **SYSTEM FOR PATELLAR ADVANCEMENT IN QUADRUPEDS**
SYSTEM FÜR PATELLASEHNENVORSCHUB BEI VIERFÜSSLERN
SYSTÈME POUR UN AVANCEMENT ROTULIEN CHEZ DES QUADRUPÈDES

(30) Priority: 31.01.2011 US 201161437980 P; 31.01.2011 US 201161437944 P
(43) Date of publication of application: 11.12.2013
(73) Proprietor: Synthes GmbH, 4436 Oberdorf (CH)
(72) Inventor: HORAN, Timothy, J., West Chester, PA 19380 (US); BUCK, Ryan, West Chester, PA 19380 (US); BORDEAUX, Jean-Noel, West Chester, PA 19380 (US)
(74) Representative: Jackson, Richard Eric
(86) International application number: PCT/US2012/023300
(87) International publication number: WO 2012/106323

(56) References cited:
- US-A1- 2006 025 776
- US-A1- 2010 076 564

## Description

### BACKGROUND

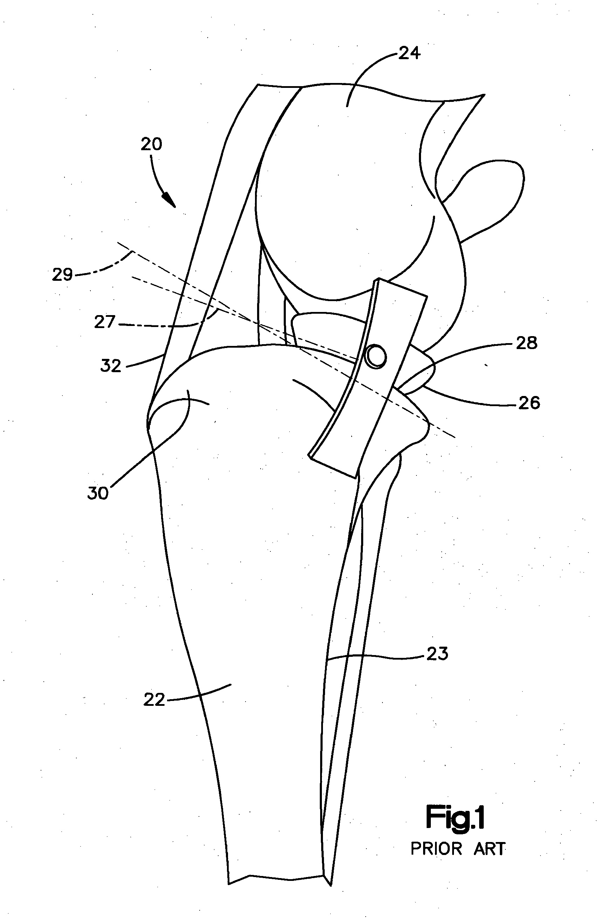

Referring to Fig. 1, the knee joint 20 of quadrupeds, such as dogs and cats, connects the tibia 22 and the femur 24 in a pivotal relationship. The knee joint 20 includes a number of stabilizing tendons and ligaments that supports the joint during anatomical function. For instance, the cranial cruciate ligament (CCL), similar to the anterior cruciate ligament in humans, bears the majority of the animal's weight, and is important to the overall stability of the knee joint 20. The CCL is attached to the tibia 22 and the femur 24, and in general prevents or limits sliding of the tibia 22 forward or cranially relative to the femur 24, and further limits internal rotation of the tibia 22 relative to the femur 24 as well as hyperextension of the knee joint 20. The knee joint 20 further includes a meniscus 26 that is disposed between the tibia 22 and the femur 24, and absorbs impact and provides a gliding surface between the femur 24 and tibial plateau 28 of the tibia 22.

The tibia 22 includes a tibial body 23 and a tuberosity 30 that extends from the tibial body 23. The patellar tendon 32 is anchored between the tuberosity 30 and the femur 24. As illustrated in Fig. 1, a line 27 extending through the patellar tendon 32 that is both normal to the patellar tendon and directed toward the tibial plateau 28 is angularly offset with respect to a line 29 that lies in the plane generally defined by the tibial plateau 28, and intersects the line 27 at a location between the patellar tendon 32 and the tibial plateau 28. Accordingly, when the CCL is damaged, which is a common injury in canines, the patellar ligament 32 does not prevent the femur 24 from travelling along the tibial plateau 28 due to tibiofemoral sheer forces when weight is applied to the injured knee join 20. As a result, damage to the CCL often results in lameness of the affected knee, damage to the meniscus 26 due to forces applied by the femur 24, and degenerative joint diseases. Furthermore, the animal can tend to overcompensate for the injured knee joint 20, which can result in rupture of the CCL of the other knee during weight-bearing anatomical function.

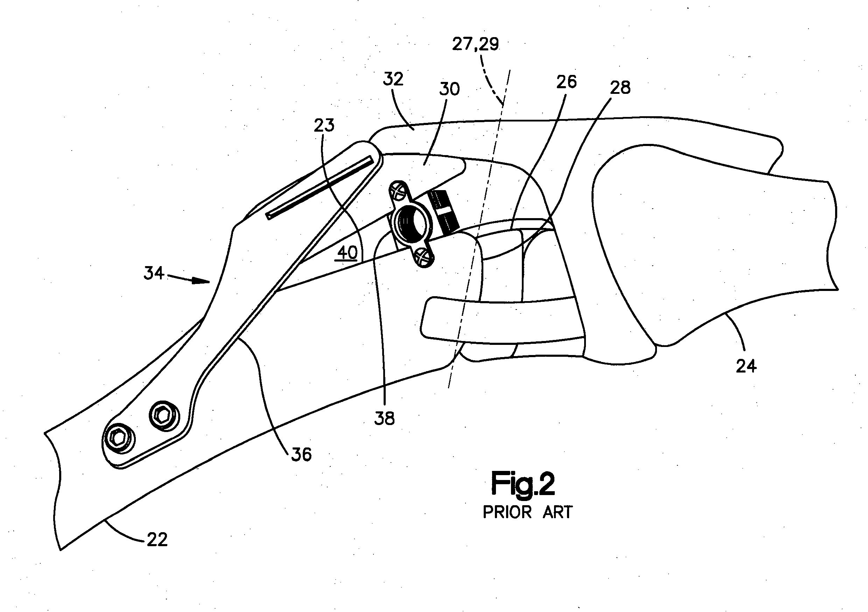

Referring also to Fig. 2, tibial tuberosity advancement (TTA) is a procedure designed to repair a knee joint 20 that has been affected by a damaged cranial cruciate ligament. Conventional TTAs include the step of performing an osteotomy cut to separate the tibial tuberosity 30 from the tibial body 23, and subsequently advancing the tibial tuberosity 30, and thus also the patellar tendon 32, cranially to a position spaced from the tibia 22 so as to define a gap 40 between the tibial tuberosity 30 and the tibial body 23. For instance, during a TTA, the tibial tuberosity 30 and the patellar tendon 32 are typically advanced such that the line 27 extending through the patellar tendon 32 that is both normal to the patellar tendon 32 and directed toward the tibial plateau 28 is also substantially parallel to, and can be coincident with, the line 29 that lies in the plane generally defined by the tibial plateau 28. Thus, the line 27 can be substantially parallel to or coincident with the plane defined by the tibial plateau 28. In general, the line 27 is more parallel to, or coincident with, the line 29, and thus the plane defined by the tibial plateau, after the TTA than before the TTA. The tibial tuberosity 30 is then fixed in the advanced position, which neutralizes the tibiofemoral sheer force when weight is applied to the knee joint 20, thereby reducing or altogether bypassing the anatomical function of the CCL.

Thus, with continuing reference to Fig. 2, a conventional TTA system 34 includes a bone plate 36 that is connected to the tibia 22 at one end, and to the advanced tibial tuberosity 30 at another end so as to provide fixation of the advanced tibial tuberosity 30 and the tibial body 23, and a spacer 38 in the form of a cage that is separate from the bone plate 36 and is disposed and connected between the advanced tibial tuberosity 30 and the tibial body 23 so as to maintain the gap 40 between the tibial tuberosity 30 and the tibial body 23 against the caudally-directed force F of the patellar tendon 32.
US 2010/076564 A1 discloses an expandable implant with first and second arms pivotally joined at apex. Each arm includes tabs that define apertures. The apertures are sized to receive bone anchors. The implant is configured to be attached to and spaced apart from tibial bone segments without the need for an additional bone plate. US 2006/025776 A1 discloses methods and systems for repositioning a portion of the tibia to align or reposition the anterior cruciate ligament. The systems include a fixation plate which is used to orient and connect the proximal end of sawn tibia to the shaft of the tibia. The plate has a first end and a second end, and a plurality of apertures; the sides connecting the first and second ends are linear.

### SUMMARY

The present invention provides a tibial tuberosity advancement implant system as recited in the claims.

### BRIEF DESCRIPTION OF THE DRAWINGS

The foregoing summary, as well as the following detailed description of the preferred embodiments of the application, will be better understood when read in conjunction with the appended drawings. In the drawings:
Fig. 1 is an illustration of a healthy knee of a canine;
Fig. 2 is a side elevation view of a conventional tibial tuberosity advancement system implanted in the knee illustrated in Fig. 1, for instance in response to an injury to the cranial cruciate ligament of the knee;
Fig. 3A is a schematic side elevation view of a tibial tuberosity advancement implant constructed in accordance with one embodiment, shown implanted in a schematically illustrated knee;
Fig. 3B is a sectional top plan view of the tibial tuberosity advancement implant illustrated in Fig. 3A, taken along line 3B-3B;
Fig. 3C is a sectional top plan view of the tibial tuberosity advancement implant similar to that illustrated in Fig. 3B, but constructed in accordance with an alternative embodiment;
Fig. 4A is a schematic side elevation view of a tibial tuberosity advancement implant similar to that illustrated in Fig. 3A, but constructed in accordance with another embodiment, shown implanted in a schematically illustrated knee;
Fig. 4B is a top plan view of the tibial tuberosity advancement implant illustrated in Fig. 4A;
Fig. 4C is a top plan view of a tibial tuberosity advancement implant similar to that illustrated in Fig. 4B, but constructed in accordance with an alternative embodiment;
Fig. 5 is a schematic side elevation view of a tibial tuberosity advancement implant constructed in accordance with another embodiment, shown implanted in a schematically illustrated knee;
Fig. 6A is a perspective view of a tibial tuberosity advancement implant constructed in accordance with another embodiment;
Fig. 6B is a perspective view of a tibial tuberosity advancement implant constructed in accordance with another embodiment
Fig. 7A is a perspective view of a tibial tuberosity advancement implant constructed in accordance with another embodiment;
Fig. 7B is an end elevation view of the tibial tuberosity advancement implant illustrated in Fig. 7A;
Fig. 8A is a perspective view of a tibial tuberosity advancement implant constructed in accordance with another embodiment;
Fig. 8B is an end elevation view of the tibial tuberosity advancement implant illustrated in Fig. 8A;
Fig. 9A is a schematic view of the tibia, showing the tibial tuberosity advanced substantially linearly along a direction substantially parallel to the tibial plateau during an osteotomy procedure;
Fig. 9B is a schematic view of the tibia, showing the tibial tuberosity advanced substantially linearly along a direction substantially parallel to the tibial plateau during an osteotomy procedure; and
Fig. 10 is a schematic view of the tibia, showing the tibial tuberosity advanced substantially curvilinearly during an osteotomy procedure.

### DETAILED DESCRIPTION

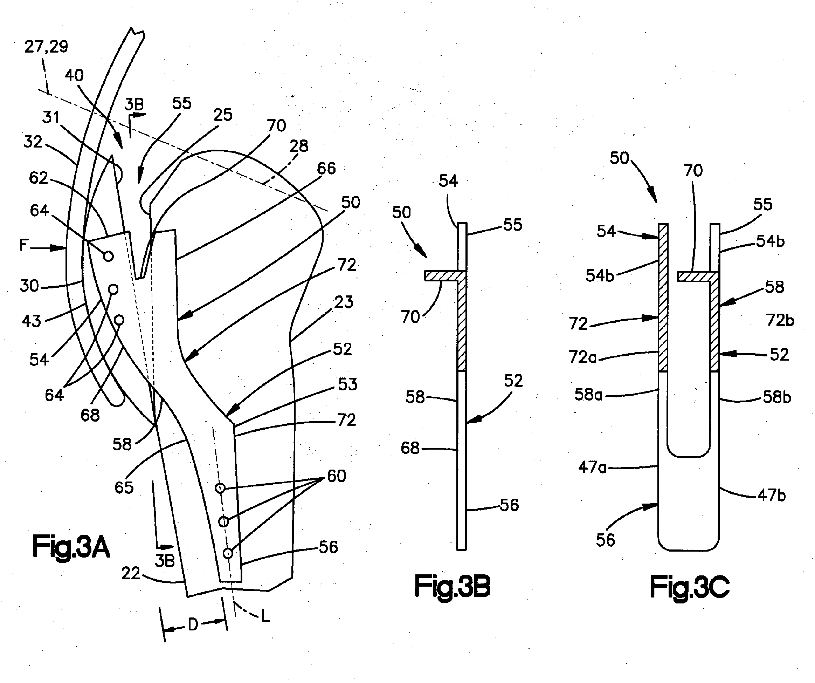

Referring to Figs. 3A-B, an implant 50, such as a tibial tuberosity advancement (TTA) implant, for a quadruped includes an implant body 52 having a proximal end 54 configured to attach to the tuberosity 30 that has been advanced along with the patellar tendon 32 a direction cranially relative to the tibial body 23 from a first position to an advanced position, an opposed distal end 56 configured to attach to the tibia 22, and an intermediate portion 58 disposed between the proximal end 54 and the distal end 56. It should be appreciated that the patellar tendon 32 is attached to the tuberosity 30 at an anatomical attachment location 43, and that the tuberosity 30 can be resected, and thus separated, from the tibial body 23 at a location distal of the attachment location 43 such that the patellar tendon 32, including the attachment location 43, is advanced along with the separateed tuberosity 30 from the first position to the advanced position. Alternatively, as will be described in more detail below, the tuberosity 30 can be separateed at a location proximal of the attachment location 43, such that the tuberosity 30 and the patellar tendon 32, but not the attachment location 43, are advanced to the advanced position.

The proximal end 54, the intermediate portion 58, and the distal end 56 can be integral with each other, and thus monolithic with each other, as desired. In accordance with the illustrated embodiment, the implant body 52 defines a leg 72 that extends proximally from the distal end 56 and includes the intermediate portion 58 and the proximal end 54. When the tuberosity 30, and the patellar tendon 32, are in the advanced position, the line 27 that extends through the patellar tendon 32 and is both normal to the patellar tendon 32 and directed toward the tibial plateau 28 is substantially parallel or coincident with the line 29 that lies in the plane generally defined by the tibial plateau 28, and thus also is substantially parallel or coincident with the plane generally defined by the tibial plateau 28. For instance, the line 27 can be parallel to or coincident with the line 29 (and thus also the plane generally defined by the tibial plateau 28), or can otherwise be more parallel or coincident with the line 29 (and thus also the plane generally defined by the tibial plateau 28) as compared to when the tuberosity 30, and the patellar tendon 32, are in the first position.

In accordance with the illustrated embodiment, the distal end 56 includes at least one attachment location such as a plurality of attachment locations illustrated as bone anchor holes 60 that are configured to receive respective bone anchors, which can be bone screws, nails, pins, or the like, so as to attach the distal end 56 to the tibial body 23, for instance to at least one of the medial and lateral sides of the tibial body 23. The distal end 56 can be contoured as desired, and adapted to conform to a medial or lateral side of the tibial body 23 to which the distal end 56 is attached. In accordance with the illustrated embodiment, the distal end 56 defines more than two bone anchor holes 60 (e.g., three bone anchor holes 60) that extend through the implant body 52. The anchor holes 60 can be substantially aligned along a longitudinal axis L that extends substantially parallel to a direction of elongation of the tibial body 23 when the implant 50 is attached to the tibial body 23 and the advanced tuberosity 30.

Though the implant 50 includes more than two bone anchor holes 60, two bone anchors can be used to fix the distal end 56 to the tibial body 23 so as to prevent rotation of the implant 50 about the distal end 56. Accordingly, all of the bone anchor holes 60 need not receive a bone anchor to fasten the implant 50 to the tibial body 23. The two bone anchors that fix the distal end 56 to the tibia prevent rotation of the implant 50 about the tibia 22. Furthermore, because the implant 50 includes more than two bone anchor holes, manual manipulation of the distal end 56 to conform to the tibia is reduced with respect to conventional implants that only define two bone anchor holes 60 that both need to be aligned with the tibia. In accordance with the illustrated embodiment, the implant 50 can be suitably attached to the tibial body 23 by fixing the distal end 56 of the implant 50 to the tibial body at only a pair, and thus at least a pair, of the plurality of the available bone anchor holes 60.

The intermediate portion 58 extends both proximally and cranially from the distal end 56 toward the proximal end 54, and thus extends along a direction that is angularly offset with respect to the longitudinal axis L. The proximal end 54 is thus both proximally and cranially spaced with respect to the distal end 56 when the implant 50 is attached to the tibial body 23 and the advanced tuberosity 30. The proximal end 54 can define a cranial region 62 that defines at least one attachment location such as a plurality of attachment locations that are aligned with the advanced tuberosity 30, and can further define a caudal region 66 that is caudally spaced from the cranial region 62 and aligned with the proximal end of the tibial body 23 (that is, the end of the tibial body 23 that is aligned with the advanced tuberosity 30 in the cranial-caudal direction). The attachment locations of the proximal end 54 can be configured as bone anchor holes 64 that are configured to receive respective bone anchors so as to attach the distal end 56 to the advanced tuberosity 30 in the manner described above with respect to the bone anchor holes 60. Thus, the proximal end 54 is configured to support the tuberosity in the advanced position. It should be appreciated that the bone anchor holes 60 and 64 can be permanent bone anchor holes, and thus configured to receive respective bone anchors for as long as the implant 50 remains implanted and attached to the tibia 22.

It should be appreciated that the implant body 52 defines a cranial edge 68 that extends along the proximal end 54, the intermediate portion 58, and the distal end 56, such that the cranial edge 68 at the proximal end 54 is cranially spaced with respect to the cranial edge 68 at the distal end 56 any distance D along the cranial-caudal direction as desired, for instance corresponding to the cranial-caudal distance between the first position of the tuberosity 30 and the advanced position of the tuberosity 30, or corresponding to the cranial-caudal distance between the tibial body 23 and the tuberosity when the tuberosity is in the advanced position. For instance, the distal end 56 is joined to the intermediate portion 58 at an elbow 53 that defines an angle between respective central axes of the distal end 56 and the intermediate portion 58 between 90 degrees and 180 degrees. Furthermore, the anchor holes 60 of the distal end 56 are spaced from the anchor holes 64 of the proximal end 54 along the cranial-caudal direction a distance sufficient to maintain the tuberosity 30 in the advanced position. Thus, the intermediate portion 58 extends between the proximal end 54 and the distal end 56 and is shaped so as to space the proximal end 54 cranially with respect to the distal end 56 an amount sufficient so as to maintain the tuberosity 30 in the advanced position.

The implant body 52 can further include a spacer 70 which can be configured as a tab that can be flexible and bent or otherwise moved from a first position that is substantially aligned with the leg 72 to a second position whereby the spacer 70 extends out, such as medially or laterally, from the plane defined by the leg 72 and into the gap 40, for instance after the implant body 52 has been attached to the tibial body 23 and the advanced tuberosity 30. Alternatively, the spacer 70 can be fixed in the second position as manufactured. It should thus be appreciated that The spacer 70 can be sized so as to define a length in the cranial-caudal direction that is substantially equal to the length of the gap 40 in the cranial-caudal direction, such that the spacer 70 abuts the caudal surface 31 of the advanced tuberosity 30 and the cranial surface 25 of the tibial body 23 when in the second position so as to maintain the gap 40 against the force F of the patellar tendon 32 that biases the advanced tuberosity 30 caudally toward the tibial body 23. Thus, the spacer 70 is configured to mechanically interfere with the advanced tuberosity 30 so as to resist forces that bias the advanced tuberosity 30 and the patellar tendon 32 caudally from the advanced position toward the first position. Accordingly, the spacer 70 maintains the gap 40 between the advanced tuberosity 30 and the tibial body 23, and supports the tuberosity 30 and the patellar tendon 32 in the advanced position so as to prevent the tuberosity and the patellar tendon from returning to the first position from the advanced position.

As illustrated in Figs. 3A-C, the implant body 52 defines a plate portion 65 that extends from, and defines, the proximal end 54, the intermediate portion 58, and the distal end 56. The spacer 70 can be integral and monolithic with the plate portion 65. For instance, the spacer 70 can be configured as a tab that is cut out of the implant body 52 so as to define a recess 55 that extends distally into the proximal end 54 of the implant body 52 such that the proximal end 54 is forked, such that the recess 55 separates the forked proximal end 54 into a first or cranial region 62 and a second or caudal region 66. The cranial region 62 of the forked proximal end 54 defines the bone anchor holes 64 that overly the advanced tuberosity 30 when the distal end 56 is attached to the tibial body 23, and the caudal region 66 of the forked proximal end 54 can overly the tibial body 23 when the distal end 56 is attached to the tibial body 23. The spacer 70 can thus extend from the proximal end 54 of the implant body 52 so as to define the recess 55, and can be flexible and bent to extend to a location between the cranial region 62 and the caudal region 66, and thus aligned with the gap 40 that is defined by and between the advanced tuberosity 30 and the tibial body 23.

It should be further appreciated that the implant 50 can be constructed in accordance with numerous alternative embodiments. In this regard, it should be appreciated that a kit of implants can include one or more of the implants 50 alone or in combination with any of the implants described herein can define various configurations, sizes, and shapes that correspond to respective sizes and shapes of the tibial body 23 and tuberosity 30, along with the length of cranial advancement of the tuberosity suitable to effectively reduce or eliminate the anatomical function of the CCL.

For instance, referring also to Fig. 3C, the distal end 56 of the implant 50 can define a medial side 47a and a lateral side 47b. The medial side 47a is configured to extend along at least a portion of the medial side of the tibial body 23 and include at least one attachment location such as a plurality of attachment locations illustrated as bone anchor holes that are configured to receive respective bone anchors, which can be bone screws, nails, pins, or the like, so as to attach the medial side 56a to the medial side of the tibial body 23 as described above with respect to the distal end 56 of Fig. 3A. Similarly, the lateral side 47b is configured to extend along at least a portion of the lateral side of the tibial body 23 and include at least one attachment location such as a plurality of attachment locations illustrated as bone anchor holes that are configured to receive respective bone anchors, which can be bone screws, nails, pins, or the like, so as to attach the lateral side 47b to the lateral side of the tibial body 23 as described above with respect to the distal end 56 of Fig. 3A. Accordingly, it can be said that the implant body 52, for instance at the distal end 56, can be configured to attach to one or both of the medial side or the lateral side of the tibial body 23.

The intermediate portion 58 and the proximal end 54 can be configured as a first and second opposed legs 72a and 72b that can be spaced along the medial-lateral direction and can be constructed as described above with respect to the leg 72 illustrated in Fig. 3A. Accordingly, each of the legs 72a and 72b can define a respective intermediate portion 58a and 58b, and a respective proximal end 54a and 54b. In accordance with the illustrated embodiment, the first and second legs 72a and 72b, respectively, extend from the opposed medial and lateral sides 47a and 47b, respectively, of the distal end 56, and extend proximally from the distal end 56. The proximal ends 54a and 54b defined by the first and second legs 72a and 72b can define at least one attachment location such as a plurality of attachment locations configured as bone anchor holes 64 that are configured to receive respective bone anchors so as to attach the proximal ends 54a-b to the medial and lateral sides of the advanced tuberosity 30, respectively, in the manner described above. At least one or both of the opposed legs 72a and 72b can include a flexible spacer 70 that is configured to extend into the gap 40 between the advanced tuberosity 30 and the tibial body 23 in the manner described above.

A method for advancing one or both of a tuberosity and a patellar tendon includes the step of cutting the tibia so as to separate the tuberosity from the tibial body. The separateed tuberosity can carry the attachment location, or the attachment location can be attached to the tibial body. Next, the separateed tuberosity is advanced cranially from the first location to the advanced location as described above. Next, any of the implant bodies as described herein can be attached to both the tibial body and the advanced tuberosity so as to fix the tuberosity in the advanced position.

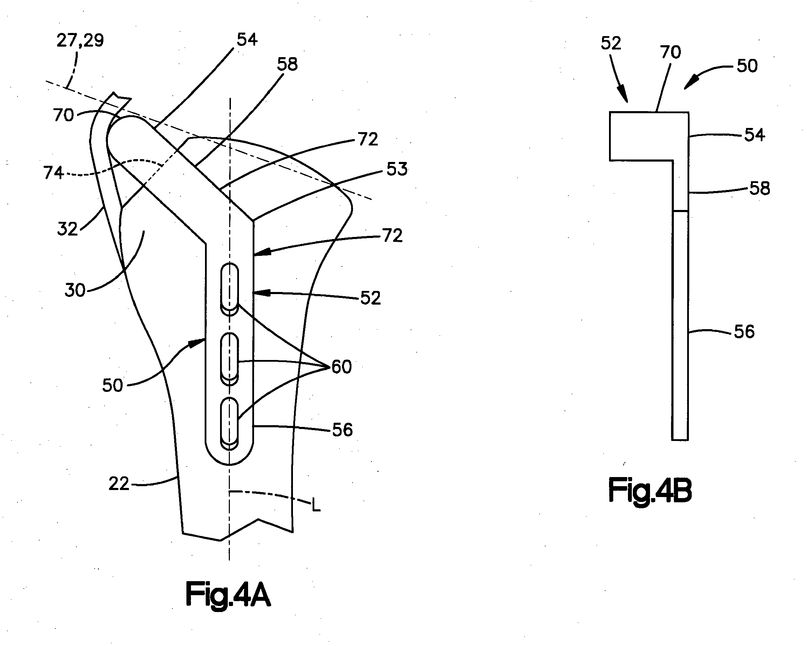

Referring now to Figs. 4A-B, the implant 50 can be configured to maintain the patellar tendon 32 in the advanced position without performing an osteotomy that separates the tuberosity 30 from the tibia 22. Thus, the tuberosity 30 can remain in the first position while the implant 50 supports the patellar tendon 32 in the advanced position and prevents the patellar tendon 32 from returning to the first position. For instance, while the distal end 56 of the implant 50 can be configured to attach to the tibia 22 in the manner described above with respect to Figs. 3A-C, the proximal end 54 can include a spacer 70 that is shaped as desired, and for instance can be configured as a barrel that can, for instance, be cylindrical. The spacer 70 can thus extend from the implant body 52 at the proximal end 54 along the medial-lateral direction which is substantially perpendicular to the cranial-caudal direction. The spacer 70 is sized to extend into the gap 40 that extends in the cranial-caudal direction between the patellar tendon 32 and the tuberosity 30 which remains integral with the tibial body 23. The spacer 70 can be flexible in the manner described above, and thus can be bent to a location so that the spacer 70 extends into the gap 40 between the tibia 22 and the advanced patellar tendon 32. Alternatively, the spacer 70 can be substantially rigid and can extend out from the plane defined by the leg 72 so as to be configured to extend into the gap 40 as manufactured. For instance the spacer 70 can abut both the cranial surface of the tibia 22, which can include the tuberosity 30, and the caudal surface of the patellar tendon 32. Accordingly the spacer 70, and thus the proximal end 54, is configured to support the tuberosity 30 in the advanced position. Accordingly, the spacer 70 is configured to maintain the gap 40 between the patellar tendon 32 and the tuberosity 30 that causes the patellar tendon 32 to be remain in the advanced position such that the line 27 extends parallel to, and can further be coincident with, the line 29 (see Fig. 3A). It should be appreciated that a notch 74 can be cut into the cranial surface of the tuberosity 30 having a geometry substantially corresponding to the outer surface of the spacer 70, such that the spacer 70 can nest within the notch 74, and also abut the caudal surface of the patellar tendon 32 as described above. The distal end 56 can be attached to the tibial body 23 at the anchor holes 60 in the manner described above such that the spacer 70, which can be integral and monolithic with the distal end 56 and thus in a fixed position relative to the distal end 56, maintains the patellar tendon 32 in the advanced position without also advancing the tuberosity 30.

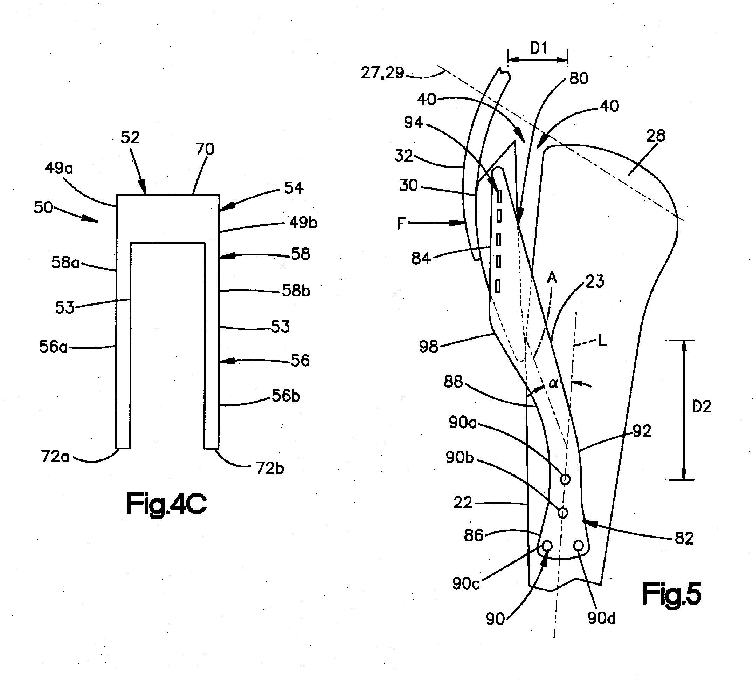

Referring to Fig. 4C, the proximal end 54 of the implant 50 can define a medial side 49a and a lateral side 49b. The medial side 49a is configured to be disposed medially with respect to the patellar tendon 32 and the tuberosity 30, and the lateral side 49b is configured to be disposed laterally with respect to the patellar tendon 32 and the tuberosity 30. The intermediate portion 58 and the distal end 56 can be configured as a first and second opposed legs 72a and 72b that can be spaced along the medial-lateral direction and can be constructed substantially as described above with respect to the leg 72 illustrated in Fig. 3A. In accordance with the illustrated embodiment, the first and second legs 72a and 72b, respectively, extend distally from the opposed medial and lateral sides 49a and 49b, respectively, of the proximal end 54, for instance of the spacer 70. The distal ends 56a and 56b defined by the first and second legs 72a and 72b can define at least one attachment location such as a plurality of attachment locations configured as bone anchor holes 60 that are configured to receive respective bone anchors so as to attach the distal ends 56a-b to the medial and lateral sides of the tibia 22, respectively, for instance to the tibial body 23 in the manner described above. The spacer 70 that extends and is connected between the opposed legs 72a and 72b is positioned to extend into the gap 40 between the advanced patellar tendon 32 and the tibia 22 and to abut the patellar tendon and the tibia 22, for instance the tuberosity 30 when the tuberosity 30 is in the first position, so as to maintain the patellar tendon 32 in the advanced position in the manner described above. It should thus be appreciated that the spacer 70 can extend and be connected between one or both of the opposed legs 72a and 72b. In accordance with the embodiment illustrated in Fig. 4C, the implant body 52 defines an elbow 53 that joins the intermediate portion 58 and the distal end 56 at each leg 72a-b so as to define an angle between respective central axes of the intermediate portion 58 and the distal end 56 between 90 degrees and 180 degrees at the elbow 53.

Referring now to Fig. 5, an implant 80 can be constructed similar to the implant 50 illustrated in Fig. 3A, but modified so as to be devoid of the spacer 70, and corresponding recess 55, and thus also devoid of the caudal region 66 spaced from the cranial region 62 at the proximal end 54. Accordingly, the implant 80 includes an implant body 82 having a proximal end 84 configured to attach to the tuberosity 30 after the tuberosity, along with the patellar tendon, has been advanced from the first position to the advanced position, along a direction cranially relative to the tibial body 23. The implant body 82 further defines a distal end 86 that is opposite the proximal end 84 and is configured to attach to the tibial body 23, and an intermediate portion 88 that extends between the proximal end 84 and the distal end 86. The proximal end 84, the intermediate portion 88, and the distal end 86 can be integral with each other, and thus monolithic with each other, as desired.

In accordance with the illustrated embodiment, the implant body 82 defines a leg 92 that extends proximally from the distal end 86 and includes the intermediate portion 88 and the proximal end 84. When the tuberosity 30, and the patellar tendon 32, are in the advanced position, the line 27 that extends through the patellar tendon 32 and is both normal to the patellar tendon 32 and directed toward the tibial plateau 28 is substantially parallel or coincident with the line 29 that lies in the plane generally defined by the tibial plateau 28, and thus also is substantially parallel or coincident with the plane generally defined by the tibial plateau 28. For instance, the line 27 can be parallel to or coincident with the line 29 (and thus also the plane generally defined by the tibial plateau 28), or can otherwise be more parallel or coincident with the line 29 (and thus also the plane generally defined by the tibial plateau 28) as compared to when the tuberosity 30, and the patellar tendon 32, are in the first position.

In accordance with the illustrated embodiment, the distal end 86 includes at least one attachment location such as a plurality of attachment locations illustrated as bone anchor holes 90 that are configured to receive respective bone anchors, which can be bone screws, nails, pins, or the like, so as to attach the distal end 86 to the tibial body 23, for instance to at least one of the medial and lateral sides of the tibial body 23. The distal end 86 can be contoured as desired, and adapted to conform to a medial or lateral side of the tibial body 23 to which the distal end 86 is attached. In accordance with the illustrated embodiment, the distal end 86 defines more than two bone anchor holes 90 (e.g., four bone anchor holes 90) that extend through the implant body 82. At least a first pair of the anchor holes 90, for instance first and second anchor holes 90a and 90b, can be spaced from each other and substantially aligned along a longitudinal axis L that extends substantially parallel to the direction of elongation of the tibial body 23 when the implant 80 is attached to the tibial body 23 and the advanced tuberosity 30. At least a second pair of the anchor holes 90, for instance third and fourth anchor holes 90c and 90d, can be offset from the longitudinal axis L, and on opposite sides of the longitudinal axis L. For instance, the third anchor hole 90c can be spaced cranially with respect to the longitudinal axis L, and the fourth anchor hole 90d can be spaced caudally with respect to the longitudinal axis L. One or both of the third and fourth anchor holes 90c and 90d of the second pair of anchor holes 90 can receive a respective bone anchor so as to attach the distal end 86 to the tibial body 23. The third and fourth anchor holes 90c and 90d can enhance the stability of the implant 80 with respect to conventional implants that only include a pair of aligned bone anchor holes at the distal end, and further includes a separate spacer (see, e.g., Fig. 2).

The intermediate portion 88 extends both proximally and cranially from the distal end 86 toward the proximal end 84, and thus extends along a direction that is angularly offset with respect to the longitudinal axis L. The proximal end 84 is thus both proximally and cranially spaced with respect to the distal end 86 when the implant 80 is attached to the tibial body 23 and the advanced tuberosity 30. The implant body 82 defines a plurality of attachment locations at the proximal end 84 that can be configured as bone anchor holes 94 that are configured to receive respective bone anchors so as to attach the distal end 86 to the advanced tuberosity 30 in the manner described above with respect to the bone anchor holes 90. The bone anchor holes 94 can be aligned along a longitudinal direction that extends substantially parallel to the longitudinal axis L. It should be appreciated that the bone anchor holes 90 and 94 can be permanent bone anchor holes, and thus configured to receive respective bone anchors for as long as the implant 80 remains implanted and attached to the tibia 22.

It should be appreciated that the implant body 82 defines a cranial edge 98 that extends along the proximal end 84, the intermediate portion 88, and the distal end 86, such that the cranial edge 98 at the proximal end 84 is cranially spaced with respect to the cranial edge 98 at the distal end 86 any distance D1 along the cranial-caudal direction as desired, for instance corresponding to the cranial-caudal distance between the first position of the tuberosity 30 and the advanced position of the tuberosity 30, or corresponding to the cranial-caudal distance between the tibial body 23 and the tuberosity when the tuberosity is in the advanced position. Furthermore, the intermediate portion 88 extends a second distance D2, for instance between and including approximately 30 mm and approximately 40 mm in the proximal-distal direction, parallel to the longitudinal axis L, from a distal end of the advanced tuberosity 30 to the center of the first, or proximal-most, anchor hole 90a at the distal end 86. The second distance D2 is greater than that of conventional TTA implants. Accordingly, the intermediate portion 88 has defines a slope relative to the proximal-distal direction that is more shallow with respect to conventional TTA implants. The slope can be defined by an angle α that is defined at the intersection of a central axis A that extends centrally through the intermediate portion 88 along a direction between the proximal and distal ends 84 and 86, respectively, and the longitudinal axis L. The angle α can be within the range of approximately 23.7° and approximately 30.2°, which is less than that of conventional TTA implants. As illustrated in Fig. 5, the implant 80 can be devoid of a spacer that is separate from the implant body 82.

Thus, the implant 80 can be devoid of a spacer that is separate from the implant body 82 and configured to secure the advanced tuberosity 30 to the tibial body 23 in the manner described. Alternatively or additionally, either or both of the implants 50 and 80 described above can include a spacer that is separate and spaced from, and thus not directly attached to, the implant body 82 and is configured to be disposed in the gap 40 between the advanced tuberosity 30 (and the advanced patellar tendon 32) and the tibial body 23 so as to resist forces that bias the advanced tuberosity 30 and the patellar tendon 32 from the advanced position toward the first position.

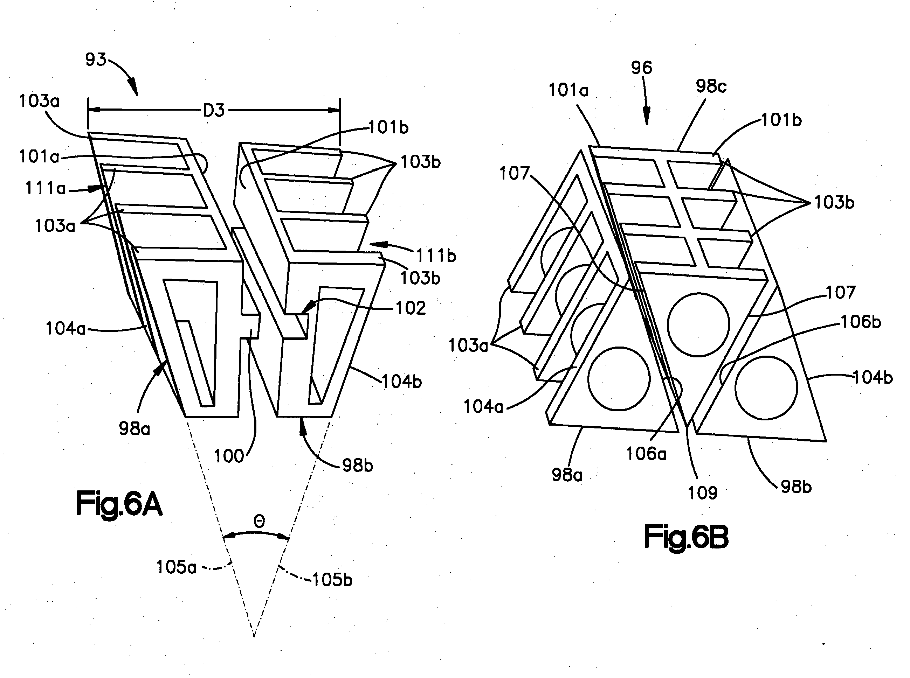

For instance, referring to Fig. 6A, the spacer 96 can include at least a pair of interlocking spacer bodies 98a and 98b. The spacer bodies 98a and 98b define respective opposed outer bone facing surfaces configured as bone contacting surfaces 104a and 104b that are positioned to face or abut the tibial body 23 and the advanced tuberosity 30, respectively, so as to resist forces that bias the tuberosity 30 toward the first position from the advanced position, thereby maintaining the gap 40 between the advanced tuberosity 30 and the tibial body 23. Thus, the spacer bodies 98a and 98b provide surface contact at the bone contacting surfaces 104a and 104b against the respective bones. The bone contacting surfaces 104a and 104b can define respective lines 105a and 105b that intersect at any suitable angle 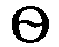 between 0 degrees and 90 degrees as desired. Thus, the bone contacting surfaces 104a and 104b can maintain the tuberosity 30 in the advanced position such that the caudal surface of the tuberosity 30 is oriented so as to define the angle 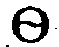 with respect to the cranial surface of the tibial body 23 when the spacer 96 is implanted in the gap 40.

The bone contacting surfaces 104a and 104b are spaced along a first direction, which can be the cranial-caudal direction when implanted into the gap 40. The spacer bodies 98a and 98b can further define respective inner surfaces 101a and 101b that are opposite and spaced from the respective bone contacting surfaces 104a and 104b along the first direction. The inner surfaces 101a and 101b can define respective engagement members that are configured to mate so as to attach the first and second spacer bodies 98a and 98b to each other. For instance, one of the spacer bodies, such as the first spacer body 98a, can include a projection 100 and the other of the spacer bodies, such as the second spacer body 98b, can define a complementary recess 102 that is configured to receive the projection 100 so as to attach the spacer bodies 98a and 98b together.

The spacer bodies 98a and 98b can each include a plurality of respective ribs 103 a and 103b that are spaced along a second direction that is substantially perpendicular to the first direction, and are oriented in a plane that is defined by the first direction and a third direction that is substantially perpendicular to the first and second directions. For instance, the second direction can extend along the medial-lateral direction and the third direction can extend along the anterior-posterior direction, or the second direction can extend along the anterior-posterior direction and the third direction can extend along the medial-lateral direction. It should be appreciated that the bone contacting surfaces 104a and 104b are sloped with respect to the inner surfaces 101a and 101b, respectively, along the third direction. The spacer body 98a can define gaps 111a between adjacent ones of the ribs 103a along the second direction, and the spacer body 98b can define gaps 111b between adjacent ones of the ribs 103b along the second direction. The outer surfaces of the ribs 103a and 103b can define the bone contacting surfaces 104a and 104b, respectively. For instance, the cranial outer surfaces of the ribs 103a can define the bone contacting surface 104a, and the caudal outer surfaces of the ribs 103b can define the bone contacting surface 104b. Accordingly, the bone contacting surfaces 104a and 104b can be discontinuous along the second direction as illustrated, or can be continuous as desired. For instance, a first portion of the bone contacting surfaces 104a and 104b can be continuous along the second direction, and a second portion of the bone contacting surfaces 104a and 104b can be discontinuous along the second direction.

The spacer 96 can include a plurality of spacer bodies 98a and 98b of different sizes such that the distance along the first direction from the outer bone contacting surfaces 104a and 104b, respectively, and the respective inner surfaces 101a and 101b can vary among different ones of the spacer bodies 98a and 98b. Accordingly, the spacer 96 can define a maximum distance D3 between the bone contacting surfaces 104a and 104b along the first direction. The maximum distance D3 of the spacer 96 can vary, for instance at less than 3mm increments, depending on which select spacer bodies 98a and 98b are interconnected so as to define the spacer 96. It should be appreciated that the spacer bodies 98a and 98b can further include a projection configured to receive at least one bone anchor hole so as to attach the spacer bodies 98a and 98b to the advanced tuberosity 30 and the tibial body 23 as desired.

Alternatively, referring to Fig. 6B, the spacer 96 can alternatively further include a third, such as a central, spacer body 98c, such that the first and second spacer bodies 98a and 98b define outer spacer bodies along the first direction, and the third spacer body 98c is connected between the outer spacer bodies 98a and 98b along the first direction. The third spacer body 98c can define opposed outer surfaces 107 that are sloped with respect to each other and converge, and are spaced along the first direction and nest within a recess 108 defined between the inner surfaces 101a and 101b of the spacer bodies 98a and 98b, which can be sloped with respect to each other and converge. Accordingly, the outer surfaces 107 of the third spacer body 98c can abut the inner surfaces 101a and 101b of the first and second spacer bodies 98a and 98b, respectively.

The first and second spacer bodies 98a and 98b can include respective engagement members carried by the respective inner surfaces 101a and 101b that are configured to mate with complementary engagement members carried by the outer surfaces 107 of the third spacer body 98c so as to attach the third spacer body 98c to the first and second spacer bodies 98a and 98b. The outer surfaces 107 of the third spacer body 98c can taper to an edged intersection 109 as illustrated, or can be truncated at variable depths such that the third spacer body 98c can be inserted to any depth relative to the first and second spacer bodies 98a and 98b as desired, thereby adjusting the distance between the opposed bone contacting surfaces 104a and 104b along the first direction. Accordingly, the position of the third spacer body 98c along the third direction can be adjusted so as to provide for a corresponding adjustment of the maximum distance along the first direction between the opposed bone contacting surfaces 104a and 104b. In accordance with the illustrated embodiment, the outer surfaces 107 are substantially parallel with the inner surfaces 101a and 101b of the first and second spacer bodies 98a and 98b.

Alternatively or additionally, a plurality of spacer bodies 98a-c can be provided having different dimensions and outer surfaces of different slopes so as to define variable distances, for instance at less than 3mm increments, between the opposed bone contacting surfaces 104a and 104b depending upon which spacer bodies 98a-c are interconnected. It should be appreciated that any number of spacer bodies 98a-c, including two, three, four, five, six, or more spacers can be stacked in the cranial-caudal direction so as to determine the distance between the outermost bone-contacting surfaces. For instance, each spacer body can define any distance as desired in the cranial-caudal direction, for example from 1mm to 3mm, such that the desired number of stacked spacers can define the desired distance between opposed bone contacting surfaces.

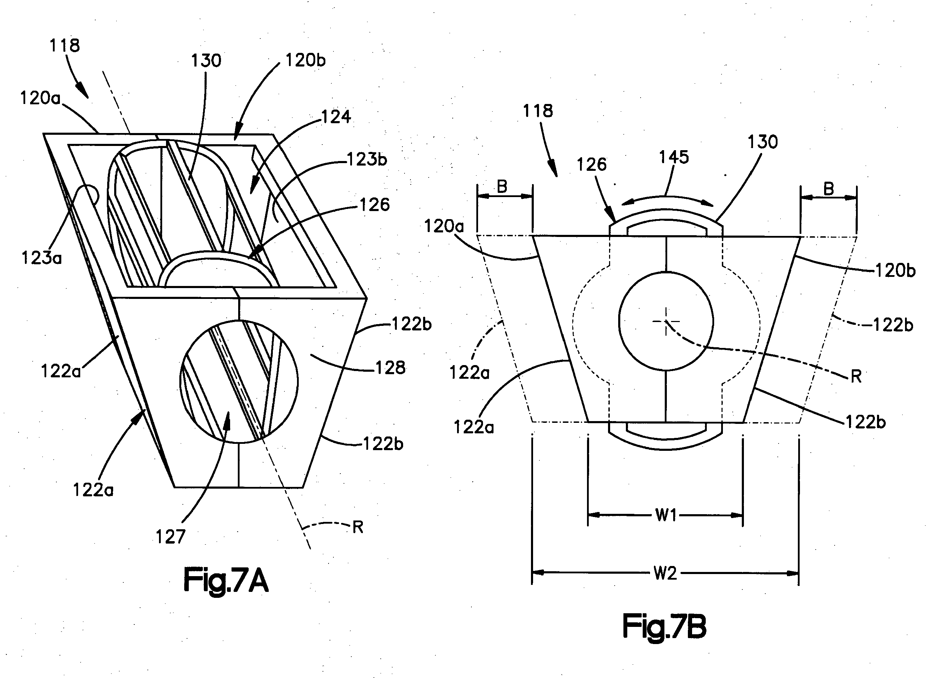

Referring to Figs. 7A-B, an expandable spacer 118 constructed in accordance with an alternative embodiment includes first and second spacer bodies 120a and 120b that are spaced from each other along the first direction. The first and second spacer bodies 120a and 120b define opposed bone facing surfaces that can further define bone contacting surfaces 122a and 122b, respectively, that are spaced along the first direction and configured to abut the tibial body 23 and the advanced tuberosity 30, respectively, when the spacer 118 is disposed in the gap between the advanced tuberosity 30 and the tibial body 23. As will be appreciated from the description below, at least one or both of the first and second spacer bodies 120a and 120b are movable, for instance, translatable relative to the other of the first and second spacer bodies 120 and 120b along the first direction so as to advance the separateed tuberosity 30 to the advanced position. The bone contacting surfaces 122a and 122b can provide surface contact against the advanced tuberosity 30 and the tibial body 23, respectively.

The spacer bodies 120a and 120b can further define respective interior surfaces 123a and 123b that are opposite the respective bone contacting surfaces 122a and 122b along the first direction, and are spaced from each other along the first direction so as to define an interior 124 that extends between the interior surfaces 123a and 123b. The spacer 118 can include an articulation member 126 that can be mounted eccentrically to one or both of the spacer bodies 120a and 120b, and is operably coupled to the spacer bodies 120a and 120b so as to bias the first and second spacer bodies 120a and 120b away from each other in the first, or cranial-caudal, direction. The articulation member 126 can be rotatable, for instance eccentrically, along an axis of rotation R that extends in the second direction. For instance, the articulation member 126 can rotate from a first rotational position whereby the articulation member 126 defines a first width W1 in the first direction, to a second rotational position that is angularly offset with respect to the first rotational position whereby the articulation member 126 defines a second width W2 in the first direction. The articulation member 126 can define an engagement member 127 that can extend through an end wall 128 of the spacer body 120, or can be accessible through the end wall 128, such that a driving tool can engage the engagement member 127 so as to rotate the articulation member 126 from the first rotational position to the second rotational position along the direction of Arrow 145

In accordance with the illustrated embodiment, the articulation member 126 defines an outer engagement surface 130 that defines a first location and a second location that is spaced further from the axis of rotation R than the first location. Accordingly, as the articulation member 126 is rotated from the first to the second position, the outer surface 130 can abut the interior surfaces 123a and 123b, so as to bias the first and second spacer bodies 120a and 120b and the corresponding bone contacting surfaces 122a and 122b away from each other along the direction of Arrow B in the first direction, thereby expanding the gap 40 between the tibial body and the separateed tuberosity 30 in the cranial-caudal direction, as illustrated in Fig. 4A. Alternatively, the outer surface 130 of the articulation member 126 can extend through the bone contacting surfaces 122a and 122b, and directly abut one or both of the tuberosity 30 and the tibial body 23, respectively, so as to advance the tuberosity 30 cranially with respect to the tibial body 23 to the advanced position when the articulation member 126 rotates to the second rotational position. In this regard, it should be appreciated that the surfaces 122a and 122b can define bone facing surfaces that do not abut the tuberosity 30.

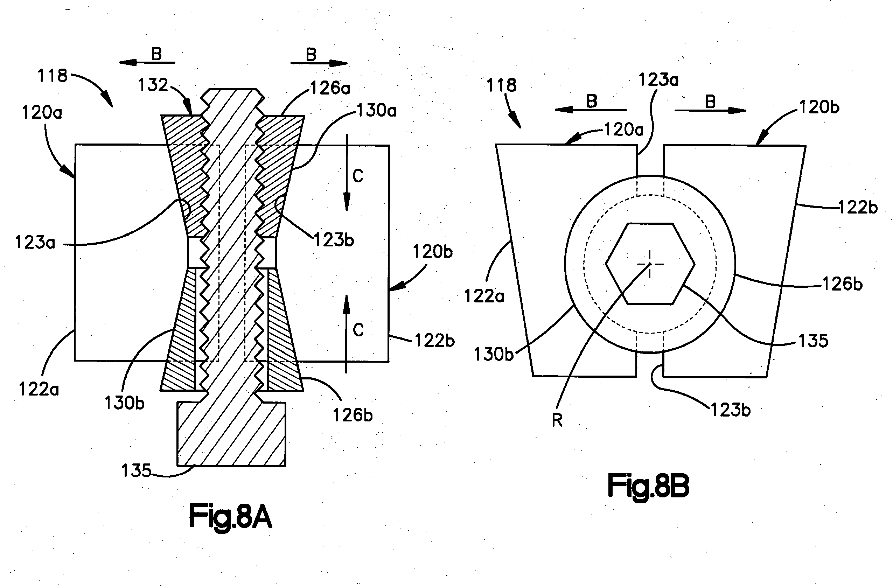

Referring to Figs. 8A-B, the spacer 118 can be constructed substantially as described above with respect to Figs. 7A-B, but includes an articulation assembly 132 that includes the articulation member 126 and an actuator 135 that is configured to actuate the articulation member from a first position to a second position that causes the spacer 118 to expand from a first position to a second position whereby the bone contacting surfaces 122a and 122b are spaced from each other further than in when the spacer is in the first position. The articulation member 126 can include a first articulation member portion 126a and a second articulation member portion 126b that threadedly receive the actuator 135, which can be configured as an actuation screw. The actuator 135 can be rotated about the axis of rotation R so as to selectively translate the articulation member portions 126a and 126b toward and away from each other along the second direction. The articulation member portions 126a and 126b can define opposed respective tapered outer engagement surfaces 130a and 130b that can be sized and shaped as desired. For instance, the outer engagement surfaces 130a and 130b can be frustoconical. The outer engagement surfaces 130a and 130b can be sloped inwardly toward the axis of rotation R as they extend along the second direction toward each other. The inner surfaces 123a and 123b can be contact each of the outer engagement surfaces 130a and 130b. For instance, the inner surfaces 123a and 123b can be sloped and in surface contact with the outer engagement surfaces 130a and 130b, respectively. Accordingly, when the articulation member portions 126a and 126b move so as to vary the distance between each other, for instance toward each other along the direction of Arrow C, the each of the outer engagement surfaces 130a and 130b rides along the inner surfaces 123a and 123b so as to bias the first and second spacer bodies 120a and 120b, and the corresponding bone contacting surfaces 122a and 122b, outward away from each other in the first direction as indicated by Arrow B.

Alternatively, the outer engagement surfaces 130a and 130b can be sloped inwardly toward the axis of rotation R as they extend along the second direction away each other. Accordingly, when the articulation member portions 126a and 126b move so as to vary the distance between each other, for instance away from each other along the direction opposite Arrow C, the respective outer engagement surfaces 130a and 130b ride along the inner surfaces 123a and 123b so as to bias the bone contacting surfaces 122a and 122b outward away from each other in the first direction as indicated by Arrow B.

The implants described above and any of the components thereof can be made from any suitable biocompatible material, such as titanium, titanium alloy, PEEK, stainless steel, or any alternative material as desired.

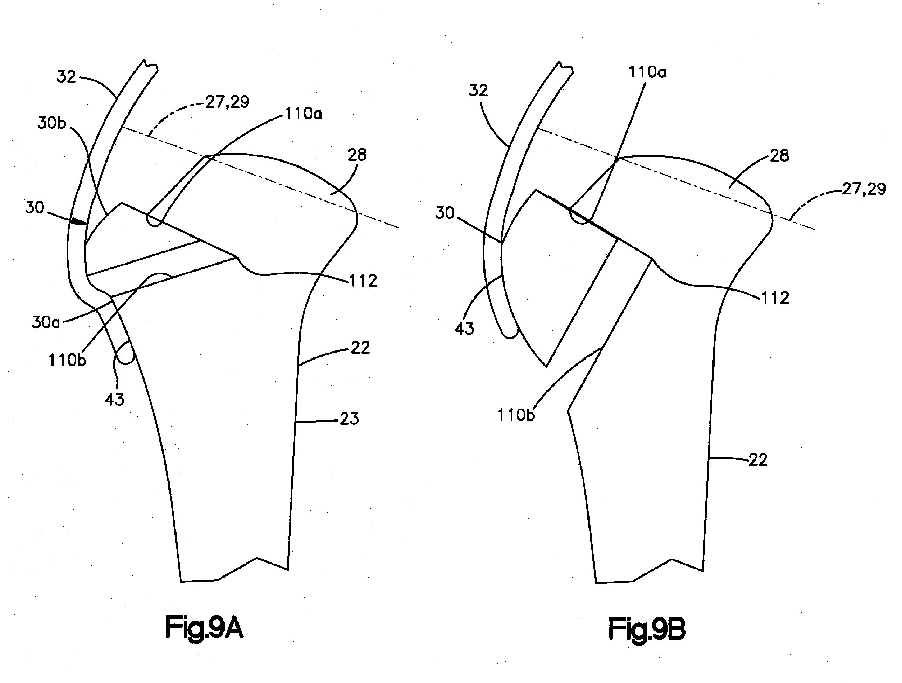

Referring now to Figs. 9A-B, an alternative method for advancing the tuberosity includes performing an osteotomy along a first or proximal cut line 110a and a second or distal cut line 110b. The first and second cut lines 110a and 110b that extend caudally from the cranial surface from the tuberosity 30 into the tibia 22. The cut lines 110a and 110b can be substantially linear and converge toward each other as they extend caudally so as to meet at a junction 112, thereby separating a separateed tuberosity 30 from the tibial body 23. The separateed tuberosity 30 can define a portion or all of the tuberosity 30. The proximal cut line 110a can extend substantially parallel to the tibia plateau 28, such that the tuberosity 30, and thus the patellar tendon 32, can be advanced cranially with respect to the tibial body 23 by translating the separateed tuberosity 30, and thus the patellar tendon 32, substantially linearly along the proximal cut line 110a from the first position to the advanced position such that the line 27 extending through the patellar tendon 32 that is both normal to the patellar tendon 32 and directed toward the tibial plateau 28 is also substantially parallel to, and can be coincident with, the line 29 that lies in the plane generally defined by the tibial plateau 28. Accordingly, the tuberosity 30 provides mechanical support for the patellar tendon, and an implant, such as the implant 50 or 80 described above, can attach to the advanced tuberosity 30 and to the tibial body 23 in the manner described above.

As illustrated in Fig. 9A, the distal cut line 110b can be located proximal to the attachment location 43 of the patellar tendon 32 to the tibial body 23, such that the distal cut line 110b is disposed between the proximal cut line 110a and the attachment location 43 Accordingly, the attachment location 43 is attached to a first portion 30a of the tuberosity 30 that remains attached to the tibial body 23 after the proximal and distal cut lines 110a and 110b have been completed so as to separate a second portion 30b of the tuberosity 30 from the tibial body 23. Thus, the attachment location 43 remains in the first position as the second portion 30b of the tuberosity 30 and the patellar tendon 32 are advanced to the advanced position. The implant bodies 52 and 82 can then be attached to the second portion 30b of the tuberosity 30 and the tibial body 23 in the manner described above. Alternatively, as illustrated in Fig. 9B, the distal cut line 110b can be located distal with respect to the attachment location 43, such that the attachment location 43 is disposed between the proximal cut line 110a and the distal cut line 110b. As a result, the tuberosity 30, along with the patellar tendon 32, including the attachment location 43, are advanced from the first position to the advanced position. The implant bodies 52 and 82 can be attached to the tuberosity 30 and the tibial body 23 in the manner described above.

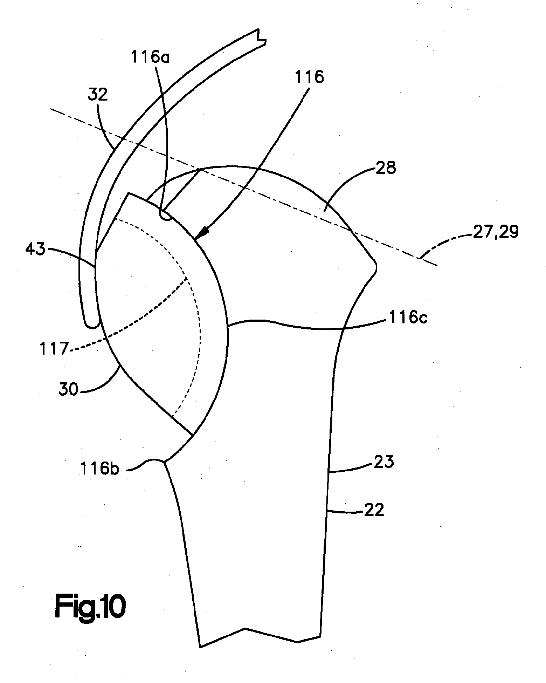

Referring now to Fig. 10, another alternative method for advancing the tuberosity includes performing an osteotomy along a cut line 116 that defines a first or proximal end 116a, a second or distal end 116b, and an intermediate portion 116c that extends between the proximal end 116a and the distal end 116b so as to separate the tuberosity 30. The proximal end 116a can be disposed proximal with respect to the attachment location 43, and the distal end 116b can be disposed distal with respect to the attachment location 43, such that the attachment location 43 is disposed between the proximal and distal ends 116a and 116b. The intermediate portion 116c can be shaped as desired. For instance, part or all of the intermediate portion 116c can be curvilinear and arc-shaped, and can define one or more substantially linear segments as desired. The tuberosity 30 can thus be rotated along the cut line 116 so as to advance at least a proximal portion of the tuberosity 30 cranially to the advanced position such that the line 27 extending through the patellar tendon 32 that is both normal to the patellar tendon 32 and directed toward the tibial plateau 28 is also substantially parallel to, and can be coincident with, the line 29 that lies in the plane generally defined by the tibial plateau 28.

Because the attachment location 43 is disposed between the proximal end distal ends 116a and 116b of the cut line 116, the attachment location 43 can be carried by the rotating separateed tuberosity 30. Alternatively, it should be appreciated that the distal end 116b of the cut line 116 can be disposed proximal with respect to the attachment location 43, such that the distal end 116b is disposed between the proximal end 116a and the attachment location 43. As a result, the patellar tendon remains fastened to the tibial body 23 at a location distal of the separateed tuberosity 30 that is rotated to the advanced position. Therefore, the tuberosity 30 and the patellar tendon 32, but not the attachment location 43, can be advanced to the advanced position.

Furthermore, one or more auxiliary cut lines can be created at a location adjacent to the cut line 116 that extend substantially parallel to the cut line 116, so as to incrementally advance the tuberosity 30. For instance, the tuberosity 30 can be rotated along the cut line 116 so as to partially advance the proximal end of the tuberosity cranially from the first position to an intermediate position that is caudal with respect to the desired advanced position. The tuberosity 30 can then be attached to the tibial body 23 so as to prevent counter-rotation along the cut line 116 due to the biasing force of the patellar tendon 32 against the tuberosity. A second cut line 117 can be created adjacent the cut line 116 and substantially parallel to the cut line 116, such that the tuberosity 30 can be rotated along the cut line 117 so as to further advance the separateed tuberosity 30 cranially from the second position to the advanced position as desired. Any one of the above-described implants 50 and 80 can then be attached to the tibial body 23 and the advanced tuberosity 30 so as to fix the tuberosity 30 in the advanced position as described above.

Although the disclosure has been described in detail, it should be understood that various changes, substitutions, and alterations can be made herein without departing from the scope of the invention as defined by the appended claims. Moreover, the scope of the present disclosure is not intended to be limited to the particular embodiments described in the specification. As one of ordinary skill in the art will readily appreciate from the disclosure of the present invention, processes, machines, manufacture, composition of matter, means, methods, or steps, presently existing or later to be developed that perform substantially the same function or achieve substantially the same result as the corresponding embodiments described herein may be utilized according to the present disclosure.

## Claims

1. A tibial tuberosity advancement (TTA) implant system configured to maintain an advanced tuberosity (30) of a quadruped in an advanced position relative to a tibial body (23), the advanced position being spaced cranially with respect to a first position when the tuberosity is integral with the tibial body, the TTA implant system comprising:
an implant body (82) including:
a distal end (86) elongate a longitudinal axis (L), the distal end defining a plurality of bone anchor holes (90), each bone anchor hole configured to receive a bone anchor so as to attach the distal end to the tibial body;
a proximal end (84) configured to receive a bone anchor so as to attach the proximal end to the advanced tuberosity, the proximal end offset with respect to the distal end in a cranial-caudal direction;
an intermediate portion (88) that extends between the proximal and distal ends along a central axis that defines an angle, α, with the longitudinal axis, wherein the intermediate portion is shaped so as to space the proximal end cranially with respect to the distal end in the cranial-caudal direction an amount sufficient so as to maintain the advanced tuberosity in the advanced position when the implant body is attached to the tibial body and the advanced tuberosity; and
an expandable spacer (96) that is separate from and not directly attached to the implant body, the expandable spacer configured to be disposed in a gap (40) defined between the advanced tuberosity and the tibial body, so as to resist movement of the advanced tuberosity toward the first position, the spacer comprising:
a first spacer body (98a) and a second spacer body (98b) spaced from the first spacer body and expandable with respect to the first spacer body in the cranial-caudal direction when the expandable spacer is disposed in the gap, the first spacer body defining a first bone facing surface (104a) configured to engage the advanced tuberosity, and the second spacer body defining a second bone facing surface (104b) configured to engage the tibial body.

2. The TTA implant system as recited in claim 1, further comprising an articulation member (126) that is operably coupled to the first and second spacer bodies, such that movement of the articulation member from a first position to a second position causes at least one of the first and second spacer bodies to move away from the other of the first and second spacer bodies.

3. The TTA implant system as recited in claim 2, wherein the articulation member (126) is rotatable about an axis of rotation, such that the second position is angularly offset with respect to the first position.

4. The TTA implant system as recited in claim 3, wherein the articulation member (126) is rotatable eccentrically about the axis of rotation.

5. The TTA implant system as recited in claim 2, wherein the articulation member (126) includes a first articulation member portion (126a) and a second articulation member portion (126b) that receive an actuator (135), such that the rotation of the actuator about an axis of rotation causes the first and second articulation member portions to bias the first and second spacer bodies away from each other.

6. The TTA implant system as recited in claim 1, wherein the angle, α, between the longitudinal axis and the central axis is within the range of approximately 23.7° and approximately 30.2°.

7. The TTA implant system as recited in claim 6, wherein the implant body (82) defines a cranial edge (98) that extends along the proximal end (84), the intermediate portion (88) and the distal end (86), wherein the cranial edge at the proximal end of the implant body is spaced from the cranial edge at the distal end of the implant body along the cranial-caudal direction a sufficient distance so as to maintain the advanced tuberosity in the advanced position when the implant body is attached to the tibial body and the advanced tuberosity.

8. The TTA implant system as recited in claim 1, wherein the spacer (96) includes an upper surface, a lower surface opposite the upper surface, the upper and lower surfaces extending between the first and second bone contact surfaces.

9. The TTA implant system as recited in claim 1, wherein the spacer (96) includes a plurality of ribs (103a, 103b).

10. The TTA implant system as recited in claim 9, wherein the plurality of ribs (103a, 103b) includes a first plurality of ribs and a second plurality of ribs, wherein the first spacer body (98a) defines the first plurality of ribs, and the second spacer body (98b) defines the second plurality of ribs.

## Patentansprüche

1. Tibial Tuberosity Advancement (TTA)-Implantatsystem, das dazu ausgebildet ist, eine vorgeschobene Tuberositas (30) eines Vierfüßlers in einer vorgeschobenen Position relativ zu einem Tibiakörper (23) zu halten, wobei die vorgeschobene Position kranial in Bezug auf eine erste Position beabstandet ist, wenn die Tuberositas mit dem Tibiakörper integriert ist, wobei das TTA-Implantatsystem aufweist:
einen Implantatskörper (82), enthaltend:
ein distales Ende (86), das sich entlang einer Längsachse (L) erstreckt, wobei das distale Ende mehrere Knochenverankerungslöcher (90) definiert, wobei jedes Knochenverankerungsloch zur Aufnahme eines Knochenankers gestaltet ist, so dass das distale Ende am Tibiakörper befestigt wird;
ein proximales Ende (84), das zum Aufnehmen eines Knochenankers gestaltet ist, so dass das proximale Ende an der vorgeschobenen Tuberositas befestigt ist, wobei das proximale Ende in Bezug auf das distale Ende in einer kranialen-kaudalen Richtung versetzt ist;
einen Zwischenabschnitt (88), der sich zwischen dem proximalen und distalen Ende entlang einer Mittelachse erstreckt, die einen Winkel α mit der Längsachse definiert, wobei der Zwischenabschnitt so geformt ist, dass das proximale Ende in Bezug auf das distale Ende in der kranialen-kaudalen Richtung mit einem ausreichenden Maß kranial beabstandet ist, um die vorgeschobene Tuberositas in der vorgeschobenen Position zu halten, wenn der Implantatskörper am Tibiakörper und der vorgeschobenen Tuberositas befestigt wird; und
einen ausdehnbaren Abstandshalter (96), der vom Implantatskörper getrennt und nicht direkt an diesem befestigt ist, wobei der ausdehnbare Abstandshalter in einem Spalt (40) ausgebildet ist, um angeordnet zu werden, der zwischen der vorgeschobenen Tuberositas und dem Tibiakörper definiert ist, so dass er einer Bewegung der vorgeschobenen Tuberositas zur ersten Position widersteht, wobei der Abstandshalter aufweist:
einen ersten Abstandshalterkörper (98a) und einen zweiten Abstandshalterkörper (98b), der vom ersten Abstandshalterkörper beabstandet und in Bezug auf den ersten Abstandshalterkörper in kranialer-kaudaler Richtung ausdehnbar ist, wenn der ausdehnbare Abstandshalter in dem Spalt angeordnet ist, wobei der erste Abstandshalterkörper eine erste dem Knochen zugewandte Oberfläche (104a) definiert, die für einen Eingriff mit der vorgeschobenen Tuberositas gestaltet ist, und der zweite Abstandshalterkörper eine zweite dem Knochen zugewandte Oberfläche (104b) definiert, die für einen Eingriff mit dem Tibiakörper gestaltet ist.

2. TTA-Implantatsystem nach Anspruch 1, des Weiteren aufweisend ein Gelenkselement (126), das funktionsfähig an den ersten und zweiten Abstandshalterkörper gekoppelt ist, so dass eine Bewegung des Gelenkselements aus einer ersten Position in eine zweite Position bewirkt, dass sich zumindest einer von dem ersten und zweiten Abstandshalterkörper von dem anderen von dem ersten und zweiten Abstandshalterkörper weg bewegt.

3. TTA-Implantatsystem nach Anspruch 2, wobei das Gelenkselement (126) um eine Drehachse drehbar ist, so dass die zweite Position winkelig in Bezug auf die erste Position versetzt ist.

4. TTA-Implantatsystem nach Anspruch 3, wobei das Gelenkselement (126) exzentrisch um die Drehachse drehbar ist.

5. TTA-Implantatsystem nach Anspruch 2, wobei das Gelenkselement (126) ein erstes Gelenkselementteil (126a) und ein zweites Gelenkselementteil (126b) enthält, die ein Stellglied (135) aufnehmen, so dass die Drehung des Stellgliedes um eine Drehachse bewirkt, dass das erste und zweite Gelenkselementteil den ersten und zweiten Abstandhalterkörper auseinander spannen.

6. TTA-Implantatsystem nach Anspruch 1, wobei der Winkel α zwischen der Längsachse und der Mittelachse im Bereich von ungefähr 23,7° und ungefähr 30,2° liegt.

7. TTA-Implantatsystem nach Anspruch 6, wobei der Implantatskörper (82) einen kranialen Rand (98) definiert, der sich entlang des proximalen Endes (84), des Zwischenteils (88) und des distalen Endes (86) erstreckt, wobei der kraniale Rand am proximalen Ende des Implantatskörpers vom kranialen Rand am distalen Ende des Implantatskörpers entlang der kranialen-kaudalen Richtung mit einem ausreichenden Abstand beabstandet ist, so dass die vorgeschobene Tuberositas in der vorgeschobenen Position gehalten wird, wenn der Implantatskörper am Tibiakörper und der vorgeschobenen Tuberositas befestigt wird.

8. TTA-Implantatsystem nach Anspruch 1, wobei der Abstandshalter (96) eine obere Oberfläche, eine untere Oberfläche gegenüber der oberen Oberfläche enthält, wobei sich die obere und untere Oberfläche zwischen den ersten und zweiten Knochenkontaktflächen erstrecken.

9. TTA-Implantatsystem nach Anspruch 1, wobei der Abstandshalter (96) mehrere Rippen (103a, 103b) enthält.

10. TTA-Implantatsystem nach Anspruch 9, wobei die mehreren Rippen (103a, 103b) eine erste Mehrzahl von Rippen und eine zweite Mehrzahl von Rippen enthalten, wobei der erste Abstandshalterkörper (98a) die erste Mehrzahl von Rippen definiert und der zweite Abstandshalterkörper (98b) die zweite Mehrzahl von Rippen definiert.

## Revendications

1. Système d'implant pour un avancement de la tubérosité tibiale (TTA) configuré pour maintenir une tubérosité (30) avancée chez un quadrupède dans une position avancée par rapport à un corps (23) tibial, la position avancée étant espacée crânialement par rapport à une première position lorsque la tubérosité fait partie intégrante du corps tibial, le système d'implant pour un TTA comportant :
un corps (82) d'implant comprenant :
une extrémité (86) distale le long d'un axe (L) longitudinal, l'extrémité distale délimitant une pluralité de trous (90) d'ancrage osseux, chaque trou d'ancrage osseux étant configuré pour recevoir un ancrage osseux de façon à fixer l'extrémité distale au corps tibial ;
une extrémité (84) proximale configurée pour recevoir un ancrage osseux de façon à fixer l'extrémité proximale à la tubérosité avancée, l'extrémité proximale étant décalée par rapport à l'extrémité distale dans une direction crânio-caudale ;
une partie (88) intermédiaire qui s'étend entre les extrémités proximale et distale le long d'un axe central qui délimite un angle α avec l'axe longitudinal, dans lequel la partie intermédiaire est formée de façon à espacer l'extrémité proximale crânialement par rapport à l'extrémité distale dans la direction crânio-caudale d'une quantité suffisante pour maintenir la tubérosité avancée dans la position avancée lorsque le corps d'implant est fixé au corps tibial et la tubérosité avancée ; et
un espaceur (96) extensible qui est séparé du corps d'implant et qui n'est pas directement fixé à ce dernier, l'extenseur extensible étant configuré pour être disposé dans un espace (40) délimité entre la tubérosité avancée et le corps tibial, de façon à résister au déplacement de la tubérosité avancée vers la première position, l'espaceur comportant :
un premier corps (98a) d'espaceur et un second corps (98b) d'espaceur espacé du premier corps d'espaceur et extensible par rapport au premier corps d'espaceur dans la direction crânio-caudale lorsque l'espaceur extensible est disposé dans l'espace, le premier corps d'espaceur délimitant une première surface (104a) en regard de l'os configurée pour venir en prise avec la tubérosité avancée, et le second corps d'espaceur délimitant une deuxième surface (104b) en regard de l'os configurée pour venir en prise avec le corps tibial.

2. Système d'implant pour un TTA selon la revendication 1, comportant en outre un élément (126) d'articulation qui est accouplé de manière fonctionnelle aux premier et second corps d'espaceur, de sorte que le déplacement de l'élément d'articulation d'une première position à une seconde position amène au moins le premier et/ou le second corps d'espaceur à s'éloigner de l'autre desdits premier et/ou second corps d'espaceur.

3. Système d'implant pour un TTA selon la revendication 2, dans lequel l'élément (126) d'articulation peut tourner autour d'un axe de rotation, de sorte que la seconde position est décalée angulairement par rapport à la première position.

4. Système d'implant pour un TTA selon la revendication 3, dans lequel l'élément (126) d'articulation peut tourner de manière excentrique autour de l'axe de rotation.

5. Système d'implant pour un TTA selon la revendication 2, dans lequel l'élément (126) d'articulation comprend une première partie (126a) de l'élément d'articulation et une seconde partie (126b) de l'élément d'articulation qui reçoivent un actionneur (135), de sorte que la rotation de l'actionneur autour d'un axe de rotation amène les première et seconde parties de l'élément d'articulation à solliciter les premier et second corps d'espaceur à l'opposé l'un de l'autre.

6. Système d'implant pour un TTA selon la revendication 1, dans lequel l'angle α entre l'axe longitudinal et l'axe central est compris entre environ 23,7° et environ 30,2°.

7. Système d'implant pour un TTA selon la revendication 6, dans lequel le corps (82) d'implant délimite une arête (98) crâniale qui s'étend le long de l'extrémité (84) proximale, la partie (88) intermédiaire et l'extrémité (86) distale, dans lequel l'arête crâniale à l'extrémité proximale du corps d'implant est espacée de l'arête crâniale à l'extrémité distale du corps d'implant le long de la direction crânio-caudale d'une distance suffisante pour maintenir la tubérosité avancée dans la position avancée lorsque le corps d'implant est fixé au corps tibial et à la tubérosité avancée.

8. Système d'implant pour un TTA selon la revendication 1, dans lequel l'espaceur (96) comprend une surface supérieure, une surface inférieure opposée à la surface supérieure, les surfaces supérieure et inférieure s'étendant entre les première et seconde surfaces de contact osseuses.

9. Système d'implant pour un TTA selon la revendication 1, dans lequel l'espaceur (96) comprend une pluralité de nervures (103a, 103b).

10. Système d'implant pour un TTA selon la revendication 9, dans lequel la pluralité de nervures (103a, 103b) comprend une première pluralité de nervures et une seconde pluralité de nervures, dans lequel le premier corps (98a) d'espaceur délimite la première pluralité de nervures et le second corps (98b) d'espaceur délimite la seconde pluralité de nervures.
